Europäisches Patentamt

European Patent Office

Office européen des brevets

(11) Veröffentlichungsnummer: **0 010 571**

A1

# (12) EUROPÄISCHE PATENTANMELDUNG

(21) Anmeldenummer: 79102290.8

(22) Anmeldetag: 05.07.79

(51) Int. Cl.³: **B 01 J 8/20**, B 01 J 8/22, B 01 J 10/00, B 01 J 14/00, B 01 J 19/24, C 12 M 1/08 // B01D11/04, C08F2/00, C08F12/00

(30) Priorität: 02.11.78 DE 2847443

(43) Veröffentlichungstag der Anmeldung: 14.05.80 Patentblatt 80/10

(84) Benannte Vertragsstaaten: AT DE FR GB

(71) Anmelder: Blenke, Heinz, Prof. Dr.-Ing., Bergwaldstrasse 40, D-7261 Gechingen (DE)

(72) Erfinder: Blenke, Heinz, Prof. Dr.-Ing., Bergwaldstrasse 40, D-7261 Gechingen (DE)

(74) Vertreter: Meyer-Dulheuer, Karl-Hermann, Dr. et al, HOECHST Aktiengesellschaft Zentrale Patentabteilung Postfach 80 03 20, D-6230 Frankfurt/Main 80 (DE)

(54) Verfahren und Vorrichtung zur Durchführung (bio-)chemischer Reaktionen und verfahrenstechnischer Grundoperationen in fluiden Systemen.

(57) Zum Durchführen (bio-)chemischer Reaktionen und verfahrenstechnischer Grundoperationen in homogenen und heterogenen fluiden Systemen werden die fluiden Systeme so geführt, daß sie während der Reaktion verschiedenartigen Strömungscharakteristiken unterliegen. Die fluiden Systeme werden zunächst in einer Schlaufe (8) geführt, deren Rohrcharakteristik zwischen idealer Rührkesselcharakteristik und realer Rohrcharakteristik einstellbar ist. Nach Verlassen der Schlaufe werden die fluiden Systeme durch ein örtlich und zeitlich vor- und/oder nachgeschaltetes Rohr (9) geführt, dessen Strömungsverlauf Rohrcharakteristik aufweist.

PROF. DR.-ING. HEINZ BLENKE   HOE 78/S 008                    /ss

Verfahren und Vorrichtung zur Durchführung (bio-)chemischer Reaktionen und verfahrenstechnischer Grundoperationen in fluiden Systemen

Anwendungsgebiete und Stand der Technik

Zur Vereinfachung werden in den Erläuterungen beispielhaft meist chemische Stoffumwandlungen (Reaktionen) und die Apparate zu ihrer technischen Durchführung (Reaktoren) betrachtet, stellvertretend zugleich für verfahrenstechnische Stoffbehandlungen (Grundoperationen) und die Apparate zu ihrer technischen Durchführung (Absorber, Kristallisator, Extraktor u.a.).

Für das Strömungs-, Misch- und Verweilzeitverhalten fluider Reaktionssysteme gibt es die Grenzfälle: Vollkommene Durchmischung (idealer Rührkesselreaktor) und Pfropfenströmung (idealer Rohrreaktor). Die realen Reaktoren nähern sich ihren jeweiligen Idealtypen mehr oder weniger an.

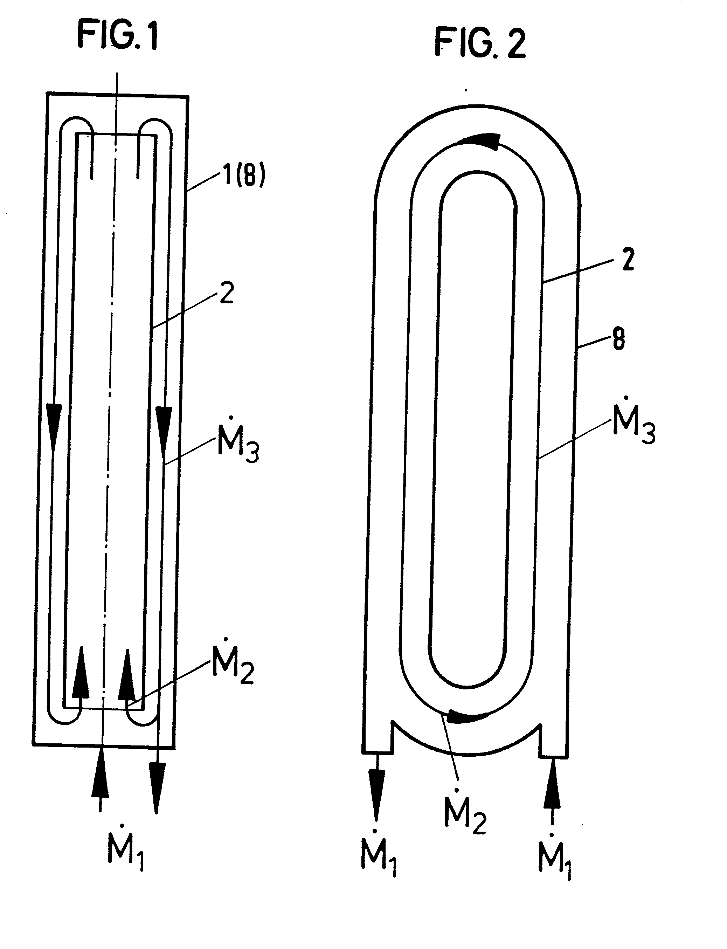

Der Schlaufenreaktor verbindet Rohr- und Rührkesselcharakteristik. Er ist dadurch gekennzeichnet, daß gemäß Fig. 1 und Fig. 2 mindestens eine definiert gelenkte Umlaufströmung erzeugt wird, die den gesamten Reaktionsraum erfaßt. Dem Umlaufstrom $\dot{M}_2$ überlagert sich der Durchlaufstrom $\dot{M}_1$ zum Gesamtstrom $\dot{M}_3 = \dot{M}_1 + \dot{M}_2$. So entsteht das Strömungsbild einer Schlaufe wie Fig. 2 deutlich zeigt. Im folgenden wird beispielhaft meist die "innere Schlaufe" gemäß Fig. 1 betrachtet.

Im Schlaufenreaktor überlagern sich zwei prinzipiell verschiedartige Mischwirkungen, nämlich

- Längsmischung im Umlauf (vor allem durch Strömungsprofil, Wirbel, Diffusion) wie im realen Rohrreaktor und

- Rückmischung durch Rezirkulation ähnlich wie im Rührkessel.

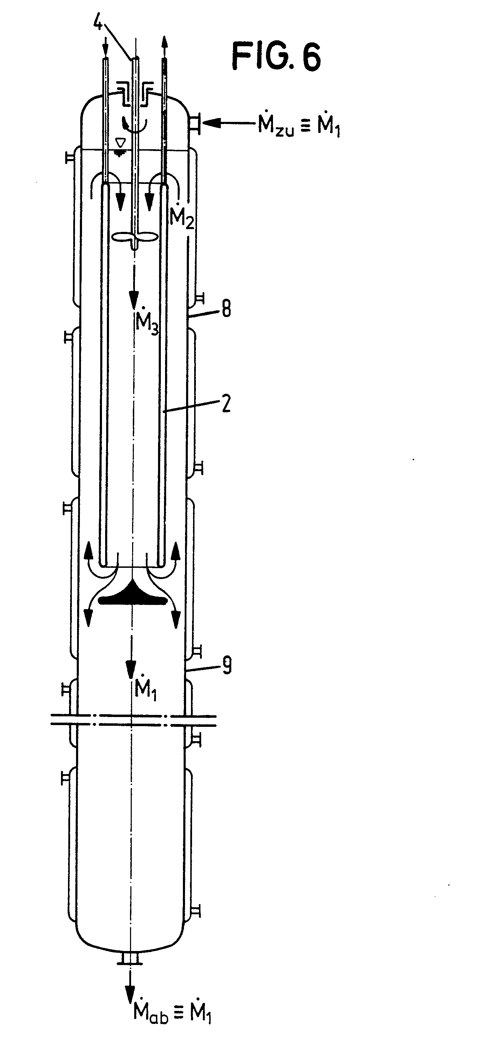

Das Mischverhalten und damit auch das Verweilzeitverhalten (bei Fließbetrieb) des Schlaufenreaktors läßt sich z.B. beim Strahl-Schlaufenreaktor gemäß Fig. 5 durch die "Umwälzzahl"

$$n_U = \frac{\dot{M}_3}{\dot{M}_1} \qquad (1)$$

folgendermaßen einstellen zwischen Rohr- und Rührkesselverhalten:

$n_U = 1$  ergibt reale Rochcharakteristik, z.B. wenn gemäß Fig. 1 im Schlaufenreaktor 1 das konzentrische Leitrohr 2 auf dem Reaktorboden aufsitzt;

$n_U \to \infty$ entspricht dem idealen Rührkessel;

$n_U \gtrsim 20$ ergibt etwa reale Rührkesselcharakteristik.

Die charakteristische gelenkte Umlaufströmung im Schlaufenreaktor (s. Figuren 1 und 2) kann erzeugt werden durch

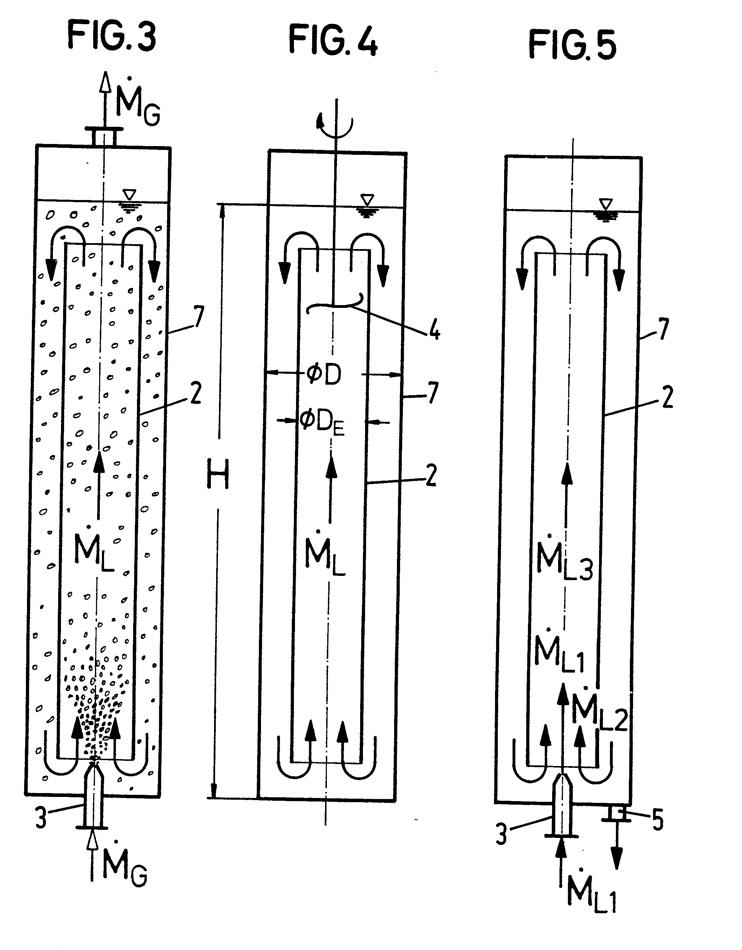

- hydrostatischen Mammutantrieb vor allem infolge unterschiedlichen Gasgehaltes (Dichtdifferenz) in der aufwärts und in der abwärts gerichteten Strömung gemäß Fig. 3, wo beispielsweise über eine Gasdüse 3, der Gasstrom $\dot{M}_G$ im Kernraum zugeführt wird. Ein Teil des Gasstromes wird über den Ringraum rezirkuliert. Durch die Dichtedifferenz in Kern- und Ringraum wird der Liquidstrom (L-Strom) $\dot{M}_L$ umgewälzt;

- hydromechanischen Propellerantrieb mittels Propeller 4, (s. Fig. 4);

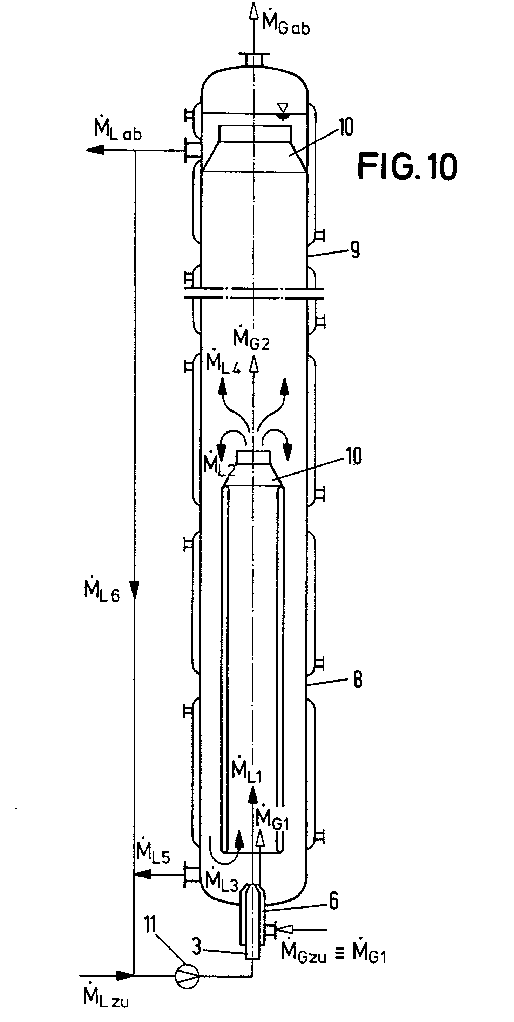

- hydrodynamischen Strahlantrieb (s. Fig. 5). Ein mit hoher Geschwindigkeit, z.B. $W_{L1} \gtrless 20\frac{m}{s}$ injizierter Liquid-Strahl (L-Strahl) $\dot{M}_{L1}$ fördert den Umlaufstrom $\dot{M}_{L2}$, vereinigt sich mit ihm zu $\dot{M}_{L3}$ und tritt durch Stutzen 5 wieder aus. Bei diskontinuierlichem Betrieb wird der austretende Liquidstrom $\dot{M}_{L1}$ über einen Pumpenkreislauf zur Düse 3 zurückgeführt, bei kontinuierlichem Betrieb enthält der L-Strahl $\dot{M}_{L1}$ in der Regel den reaktionstechnisch bedingten L-Massendurchsatz $\dot{M}_{Lzu}$ (s. auch Figuren 10 bis 12).

Die Richtung der charakteristischen gelenkten Umlaufströmung kann umgekehrt werden z.B., indem in Fig. 3 die Gaszufuhr im Ringraum erfolgt, in Fig. 4 der Propeller 4 nach unten fördert und in Fig. 5 der L-Strahl im Kernraum von oben nach unten injiziert wird. Im folgenden wird beispielhaft immer nur eine Umlaufrichtung angegeben.

Die Vorteile von Schlaufenreaktoren sind:

- Gleichmäßige Verteilung (Durchmischung) aller Komponenten im gesamten Reaktionsraum der Schlaufe durch definiert gelenkte Umlaufströmung z.B. mit Hilfe des Leitrohrs 2 in Fig. 1;

- gezielte Einstellung des Misch- und Verweilzeitverhaltens bei Fließbetrieb z.B. durch $n_U$ (1) gemäß Fig. 5 oder des Mischverhaltens bei Chargenbetrieb z.B. durch die Umlaufrate

$$\gamma_U = \frac{\dot{M}_L}{\dot{M}_R} \qquad (2)$$

(mit MR als Liquidmasse im Schlaufenreaktor) gemäß Fig. 4;

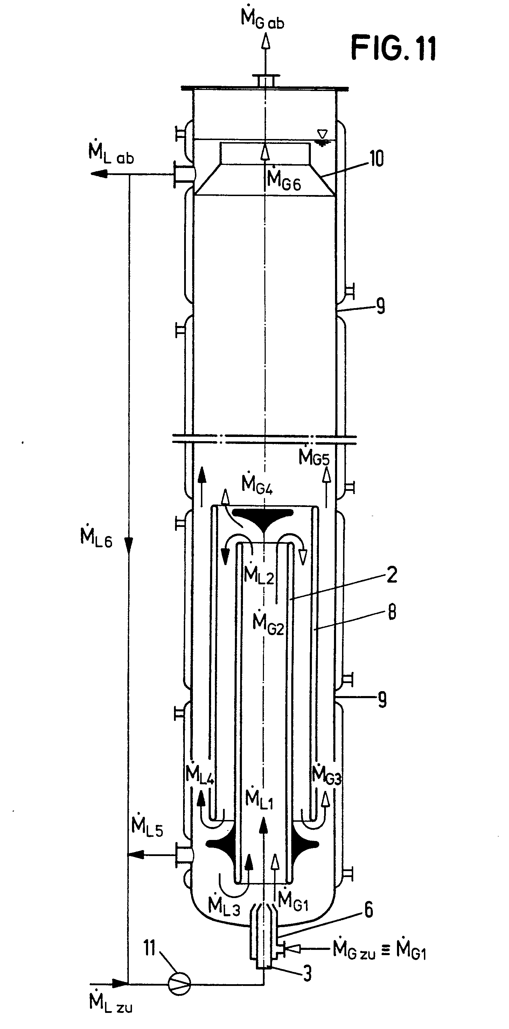

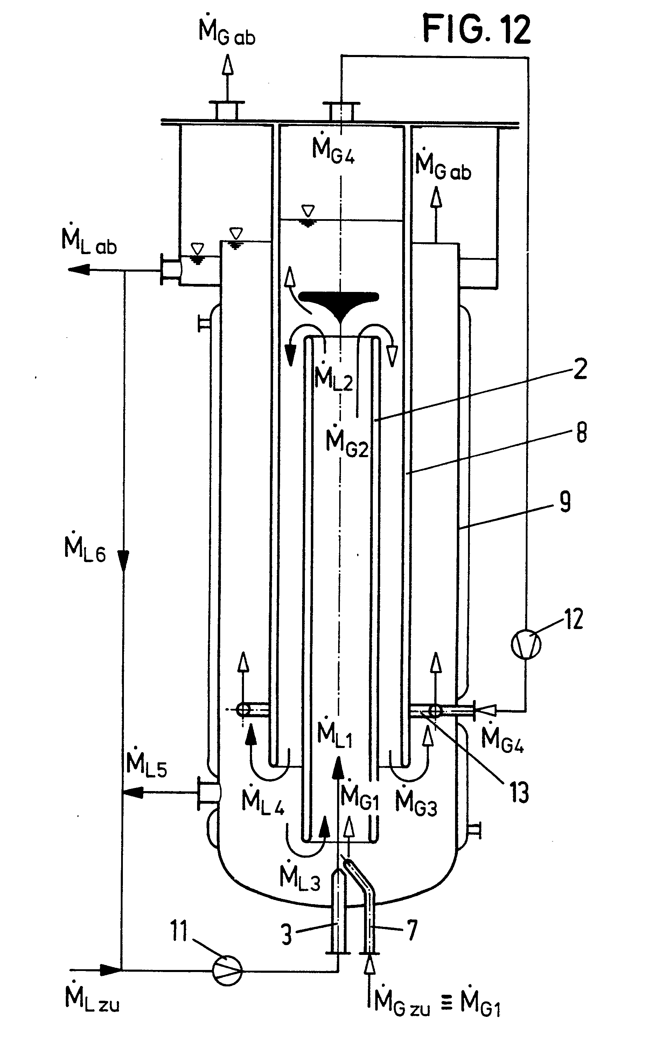

- feine Zerteilung (Primärdispergierung) des eintretenden Gases $\dot{M}_{G1}$, das beispielsweise gemäß den Figuren 10 und 11 über eine Ringdüse 6 oder gemäß Fig. 12 über ein schräges Begasungsrohr 7 an den injizierten Liquidstrahl $\dot{M}_{L1}$ herangeführt werden kann; gleicherweise ist feine Dispergierung nicht mischbarer Flüssigkeiten und agglomerierter Feststoffpartikel möglich;

- wirkungsvolle Redispergierung aller im Umlauf rezirkulierten dispersen Phasen (Gasblasen, Tropfen, Partikelagglomerate);

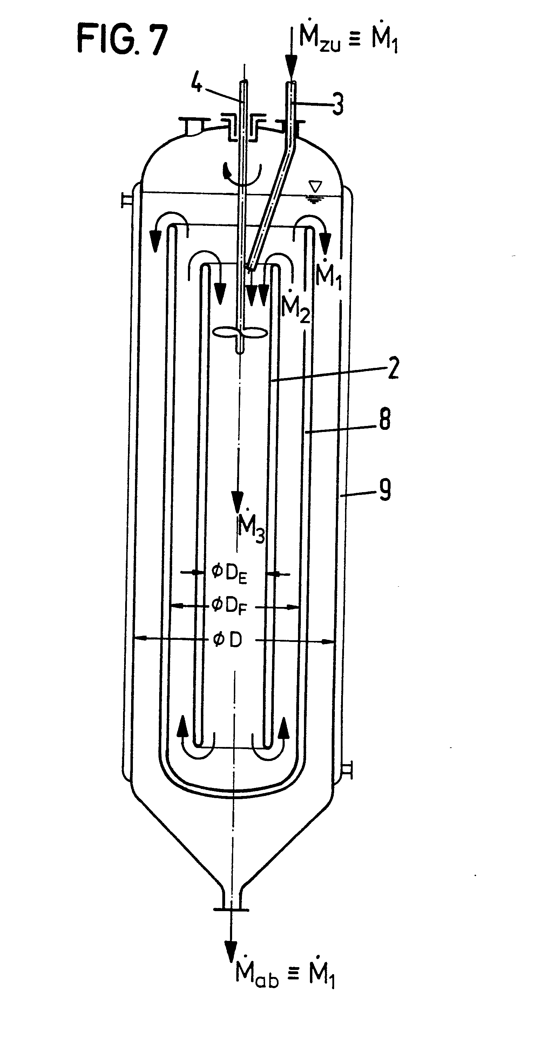

- große spezifische Wandkühlflächen infolge der schlanken Bauform mit Höhe:Durchmesser $\lesssim 5$ (s. Fig. 4) und der Möglichkeit, zusätzlich die Leitvorrichtungen z.B. gemäß Fig. 7 als Zusatzkühlflächen auszuführen /6/;

- geringer spezifischer Leistungsbedarf $\frac{P}{V}$ $/^{-}\frac{kw}{m^3}^{-}/$ für die Verteilwirkung (Mischung) infolge gelenkter verlustarmer Strömung und für die Zerteilwirkung (Dispergierung) insbesondere durch den injizierten Liquidstrahl /9,10/.

Darstellung der Erfindung mit 2 Beispielen

Beispiel 1:    Chemische Reaktion im Liquidsystem
               (Styrolpolymerisation)

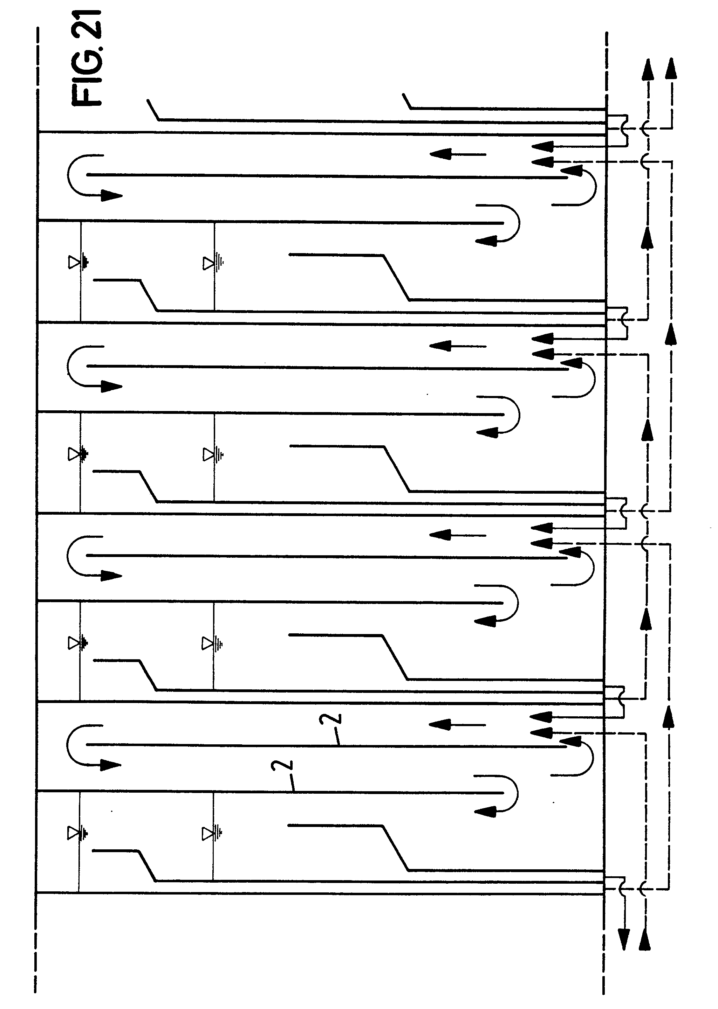

Der gute Verteileffekt des Schlaufenreaktors im gesamten Reaktionsraum bei intensiver Rezirkulation kann bei Liquidsystemen ohne Gasphase, z.B. der Styrolpolymerization, nachteilig sein, weil er Hochpolymeres rezirkuliert und mit Niederpolymerem und Monomerem vermischt. Diese Polymerisationen führt man deshalb meist in hintereinander geschalteten Reaktoren gemäß Fig. 21 /11/ durch:

- Vorpolymerisation bis zu einem Umsatz von etwa 40 % in parallel geschalteten Rührkesseln. Hier ist gute Durchmischung wegen der hohen Reaktionsgeschwindigkeit sowohl hinsichtlich des stofflichen Umsatzes als auch der abzuführenden großen Reaktionswärme erwünscht.

- Nachpolymerisation bis zu praktisch 100 %igem Umsatz in einem "Turmreaktor" mit Rohrcharakteristik, also ohne Rückmischung.

Die Erfindung sieht demgegenüber die Kombination eines Schlaufen- 8 und eines Rohrreaktors 9 z.B. gemäß Fig. 6 und 7 vor. Der Schlaufenreaktor 8 bietet für die Vorpolymerisation gegenüber dem Rührkessel folgende Vorteile:

- Große spezifische Kühlfläche durch schlanke Bauform und Zusatzkühlung mit Leitrohren 2, so daß bei $\frac{H}{D} \approx 5$ die maximale Größe, bis zu der die entstehende Wärme noch abgeführt werden kann, etwa 27 mal so groß ist wie beim konventionellen Rührkessel mit $\frac{H}{D} \approx 1$ /6/. Mehrere parallel geschaltete Rührkesselreaktoren können also durch einen Schlaufenreaktor ersetzt werden.

- Der aus dem Schlaufenreaktor in den Rohrreaktor eintretende Durchlaufstrom $\dot{M}_1$ ist polymereinheitlicher als beim Rührkessel, weil alle Teilmassen mindestens einen halben Umlauf (Fig. 6) bzw. einen vollen Umlauf (Fig. 7) passieren müssen und die mittlere Umlaufzeit gemäß (2) und Fig. 7 eingestellt werden kann zu

$$\overline{t}_U = \frac{\dot{M}_R}{\dot{M}_3} = \gamma_U^{-1} \qquad (3)$$

Diese mittlere Umlaufzeit verhält sich zur mittleren Verweilzeit des Durchsatzes $\dot{M}_1$ im Schlaufenreaktor

$$\overline{t} = \frac{\dot{M}_R}{\dot{M}_1} \qquad (4)$$

wie

$$\frac{\overline{t}_U}{\overline{t}} = \frac{\dot{M}_1}{\dot{M}_3} = n_U^{-1}$$

- 6 -

0010571

Die Polymereinheitlichkeit beim Austritt aus dem Schlaufenteil 8 kann also mit $n_U$ eingestellt werden und ist in jedem Fall besser als bei Verwendung von Rührkesseln, bei denen zugeführtes Monomeres direkt zum Auslaufstutzen gelangen kann.

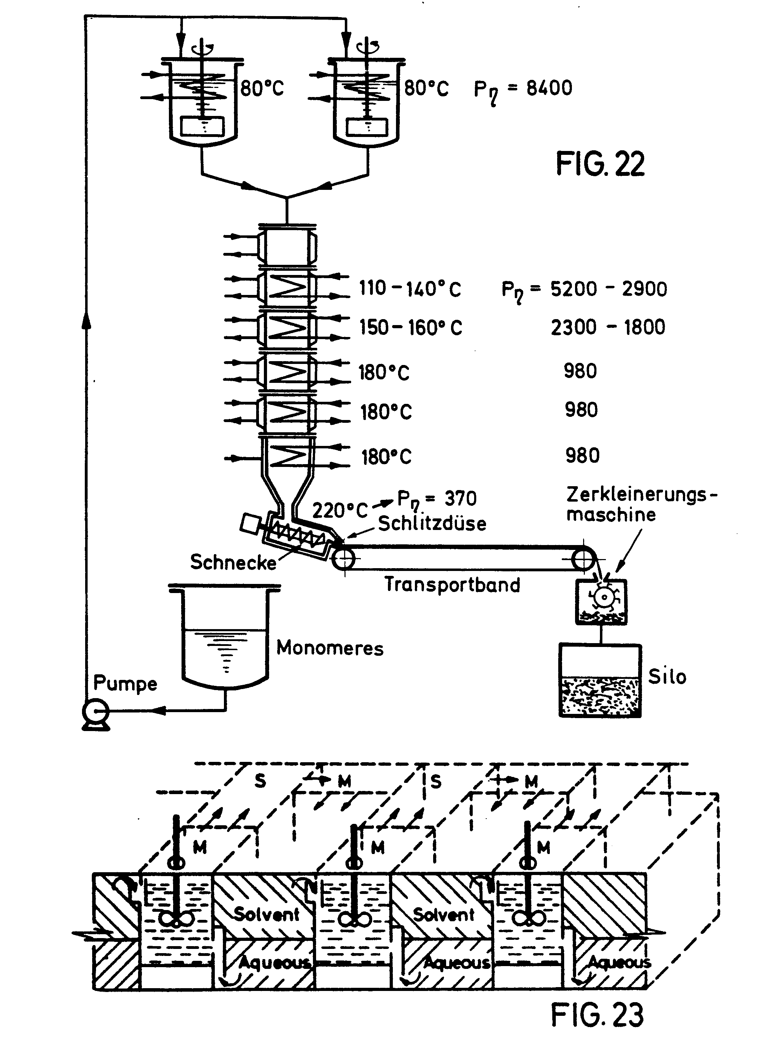

- Da für die Vorpolymerisation ein Schlaufenreaktor 8 ausreicht und dieser dieselbe schlanke Bauform hat wie der nachgeschaltete Turmreaktor 9, lassen sich beide Reaktoren zu einem Gesamtreaktor vereinigen, wie das beispielsweise die Figuren 6 und 7 zeigen. Das führt zu wesentlich einfacherer Bauform gegenüber den konventionellen Anlagen gemäß Fig. 22 und ermöglicht eine beträchtliche Senkung der Investitionskosten.

Der Durchmesser des unteren Rohrreaktorteils in Fig. 6 kann je nach benötigter Verweilzeit und angestrebter Bauhöhe auch von dem des oberen Schlaufenteils verschieden sein. Diese Reaktorbauform ähnelt der von Rektifizierkolonnen und eignet sich besonders - wie diese - für Freiluftbauweise.

Der Rohrteil 9 ist hier unter dem Schlaufenteil 8 angeordnet, weil der Styrol-Durchsatz mit zunehmendem Polymerisationsgrad spezifisch schwerer wird, also auch die Dichtedifferenz ihn im gewollten Strömungssinn abwärts treibt. Die Anordnung kann aber grundsätzlich auch umgekehrt ausgeführt werden, wenn das günstiger ist.

Dieselbe verfahrensmäßige Koppelung von Schlaufen- und Rohrcharakteristik läßt sich durch die Ausführung Fig. 7 verwirklichen. Dabei ist der Rohrteil 9 des Reaktors um den Schlaufenteil 8 herum angeordnet. Diese Ausführung ergibt bei gleichem Gesamtreaktorvolumen niedrigere Gesamtbauhöhe und noch größere spezifische Kühlfläche als die Ausführung gem. Fig. 6.

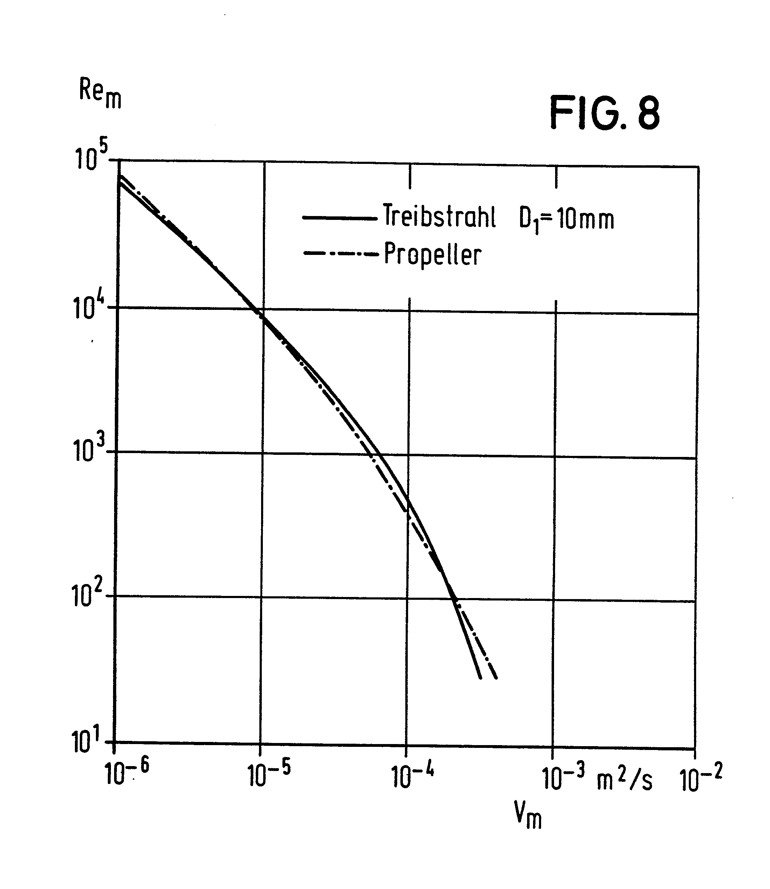

- In beiden Ausführungsarten des kombinierten Schlaufen-Rohr-Reaktors läßt sich der Umlauf im Schlaufenteil auch hydrodynamisch mit Treibstrahl oder hydrostatisch mit Inertgas antreiben. Fig. 8 zeigt Versuchsergebnisse für Treibstrahl- und Propellerantrieb mit höherviskosen Medien /7/, wie sie im gewählten Beispiel der Polymerisation vorliegen. Als Maß für die erzeugte Umlaufintensität im Schlaufenteil eignet sich besonders die mittlere Umlauf-Reynoldszahl /1/, wie Fig. 7 veranschaulicht:

$$Re_m \equiv \frac{W_m \cdot D_F}{\gamma_m} \qquad (6)$$

Dabei ist eine mittlere Umlaufgeschwindigkeit $W_m$ der Flüssigkeit so definiert /1/, daß sie die Strömungen im Kern- und Ringraum erfaßt, aber dennoch unabhängig vom Durchmesser $D_E$ des Leitrohrs ist, und zwar gemäß Fig. 7 mit $n_U$ (1) zu:

$$W_m \equiv 2 \frac{\dot{M}_3}{\mathcal{S}_m \cdot \frac{\pi}{4} \cdot D_F^2} = \frac{8 \dot{M}_1}{\mathcal{S}_m \cdot \pi \cdot D_F^2} \cdot n_U \qquad (7)$$

Aus Fig. 8 ist zu entnehmen, daß im untersuchten Bereich der kinematischen Zähigkeit $\gamma_m$ beide Antriebsarten etwa gleiche Umlauf-Reynoldszahlen ergeben. Strahlantrieb bietet gegenüber Propellerantrieb die Vorzüge, daß der Reaktor keine bewegten Bauteile aufweist, dadurch z.B. keine Abdichtungsprobleme an Wellendurchführungen und keine Schwingungen verursacht.

Unabhängig von der Antriebsart erfordert der Schlaufenreaktor aufgrund seiner definierten Strömungsführung nur etwa die Hälfte bis ein Viertel des spezifischen Leistungseintrags eines üblichen Rührkesselreaktors, in dem viel Leistungen nutzlos in Wärme dissipiert wird. Die Erfindung ermöglicht also auch eine beträchtliche Senkung des Energieverbrauchs und damit der Betriebskosten.

Beispiel 2:

Biochemische Reaktion gekoppelt mit Absorption und Diffusion im Gas-Liquid-Solid System (Biomasse-Produktion)

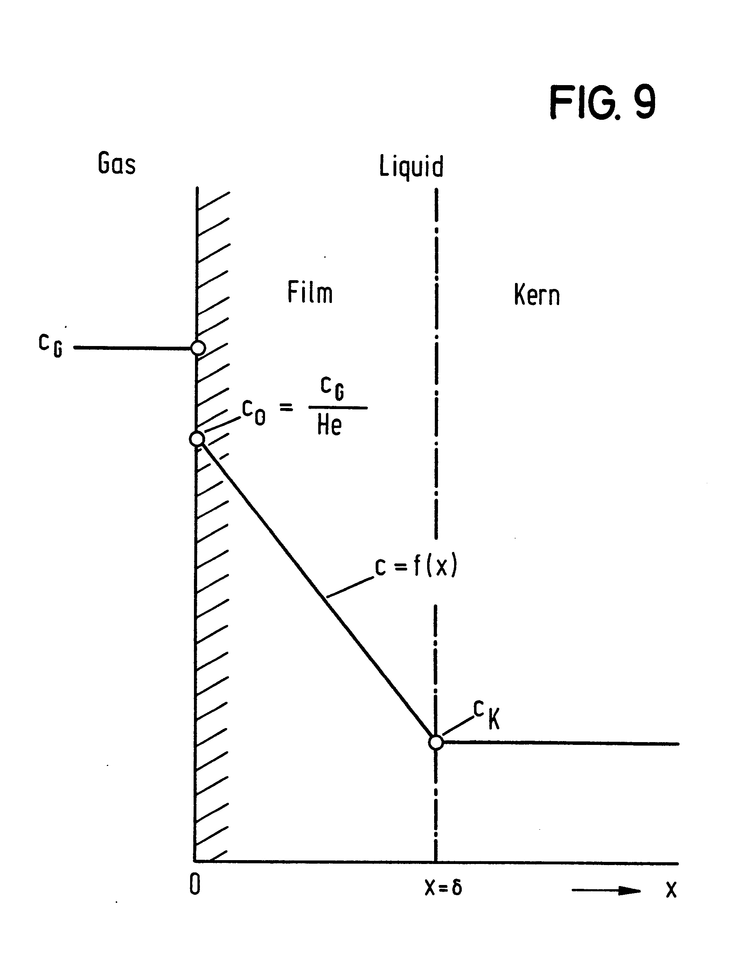

Auch für Reaktionen in heterogenen Systemen z.B. Gas-Liquid-Solid (G-L-S)-Systemen bietet die Kombination von Schlaufen- und Rohrcharakteristik bedeutende Vorteile, wie am Beispiel der Biomasse-Produktion zur Erzeugung von Single Cell Protein (SCP) gezeigt sei /8/. Eine der schwierigsten verfahrenstechnischen Aufgaben ist dabei, den hohen $O_2$-Bedarf der in der wässrigen "Kulturbrühe" (L-Phase) suspendierten Einzeller wie Hefen oder Bakterien (S-Phase) von $\dot{m}_{O_2} \approx 8$ kg $O_2$ je $m^3$ Brühe und Stunde durch Belüftung des Systems (G-Phase) einzutragen. Der $O_2$-Transport von den Luftblasen an die Zellen wird im wesentlichen bestimmt durch die Diffusion im Liquidfilm an der Gas-Liquid-Phasengrenzfläche entsprechend dem 1. Fickschen Diffusionsgesetz (s. Fig. 9) /6/:

$$\dot{m}_{O_2} = K_L \cdot a \cdot \overline{\Delta}\, c_{O_2} \qquad (8)$$

$m_{O_2} \approx 8 \dfrac{kg}{m^3 \cdot h}$ : Spezifischer $O_2$-Bedarf

$K_L \approx 1{,}6 \dfrac{m}{h}$ : $O_2$-Transferkoeffizient

$a_L \equiv \dfrac{A\underline{/}^-m^2\underline{\_}7}{V_L\underline{/}^-m^3\underline{\_}7} \approx 2000\ m^{-1}$ : Spezifische G-L-Phasengrenzfläche bezogen auf Liquidvolumen $V_L$ der Kulturbrühe

$\overline{\Delta}\, c_{O_2} = c_0 - c_K \approx 2{,}5 \cdot 10^{-3} \dfrac{kg}{m^3}$ : Treibendes Konzentrationsgefälle für den $O_2$-Transfer

$c_0 = \dfrac{c_G}{H_e}$ : Henry'sches Absorptionsgleichgewicht

Hier sei nur der geschwindigkeitsbestimmende Schritt der Diffusion des aus der G-Phase (Luft) in der L-Phase (Was-

ser) absorbierten $O_2$ durch den L-Film gemäß der Filmtheorie betrachtet, also eine verfahrenstechnische Grundoperation. Die damit gekoppelte Reaktion kann jede in der L-Phase stattfindende (bio-)chemische Stoffumwandlung mit Beteiligung eines gasförmigen Reaktanden sein, wie z.B. auch die Oxidation von Natriumsulfit zu Natriumsulfat, die Essigsäure oder die Acrylsäure-Synthese. Enscheidend sind die erforderlichen Gaseinträge (z.B. $\dot{m}_{O_2}$) je $m^3$ Liquid und Zeiteinheit, d.h. die in (8) aufgeführten Einflußgrößen, die von der Verteilungs- und Zerteilungswirkung des Reaktor abhängen. Der Schlaufenreaktor hat diesbezüglich im wesentlichen folgende Vorzüge:

- Er schafft bei Strahlantrieb die große spezifische G-L-Phasengrenzfläche $a_L \approx 2000 \ m^{-1}$ mit dem geringen spezifischen Leistungseintrag $\frac{P}{V_L} \approx 2 \ \frac{kw}{m^3}$, während andere Reaktortypen dafür die zwei- bis vierfache Leistung benötigen /9,10/. Das liegt an der ausgezeichneten Dispergierung des Gases durch den injizierten L-Strahl gemäß den Figuren 10 bis 12, der hier also sowohl die Zerteilung (Gasdispergierung) als auch die Verteilung (Umlaufantrieb) bewirkt.

- Die intensive Durchströmung und Mischung im gesamten Reaktionsraum des Schlaufenteils aufgrund der definiert gelenkten Umlaufströmung gewährleistet, daß im betrachteten Beispiel überall der benötigte $O_2$-Nachschub sichergestellt ist und keine Überkonzentration an Methanol (Substrat), womit die Mikroorganismen bevorzugt "gefüttert" werden, auftritt. Die Konzentrationen des $O_2$ sollten etwa 1 ppm nicht unterschreiten und die des Methanols etwa 1 % nicht überschreiten.

- Der $O_2$-Transferkoeffizient $K_L$ hat praktisch denselben Wert wie im intensiv durchmischten Begasungsrührkessel und liegt wesentlich über dem von Blasensäulen /9/.

- Die große spezifische Kühlfläche und der hohe Wärmeübergangskoeffizent $\alpha_i \approx 3 - 4 \frac{kw}{m^2 \cdot K}$ im Schlaufenreaktor 8 /12/ ermöglichen es, den sehr hohen spezifischen Wärmestrom von $\dot{q} \approx 40$ kw je $m^3$ Kulturbrühe bei den sehr kleinen Temperaturdifferenzen von $\Delta T \approx 5 - 10^{\circ}C$ direkt ohne äußeren Kühlkreislauf abzuführen bis zu Reaktorgrößen von $V_R \approx 600 \ m^3$ /8/.

Der Schlaufenreaktor hat aber mit seiner Rezirkulation zwei beträchtliche Nachteile (ebenso wie der Begasungsrührkessel) gegenüber einem Reaktor vom Rohrtyp (im Fall des G-L-Systems z.B. einer Blasensäule) den Gas und Flüssigkeit praktisch ohne wesentliche Rückmischung durchströmen:

- Für das treibende Konzentrationsgefälle des $O_2$-Transfers (s. Fig. 9)

$$\overline{\Delta c} = c_O - c_K \qquad (9)$$

gilt bei Rohrcharakteristik des Gasdurchsatzes für $c_O$ das logarithmische Mittel von $c_{O\alpha}$ am Gaseintritt und $c_{O\omega}$ am Gasaustritt /9,10/, also

$$c_O \equiv \overline{c_{O_{ln}}} = \frac{CO_\alpha - CO_\omega}{l_n \frac{c_{O\alpha}}{c_{O\omega}}} \qquad (10)$$

Beim idealen Rührkessel mit vollkommener Durchmischung auch der Gasphase gilt hingegen

$$c_O \equiv c_{O\omega} \qquad (7)$$

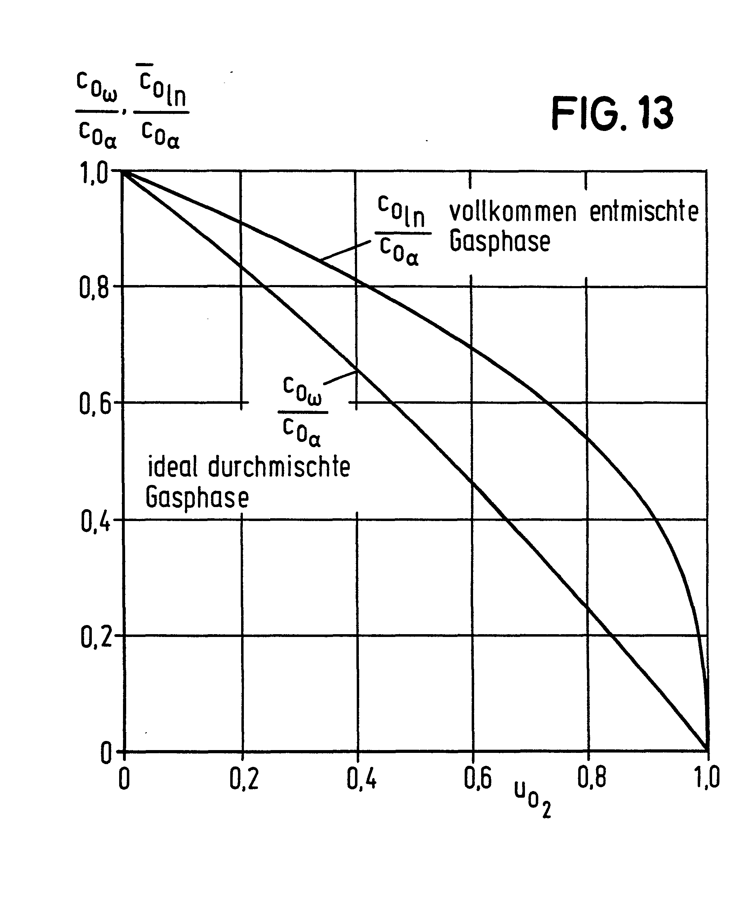

Fig. 13 zeigt, daß mit zunehmendem $O_2$-Umsatz $u_{O_2}$ der durchströmenden Luft $\overline{c_{O_{ln}}}$ wesentlich größer wird als $c_{O\omega}$ (hier beide bezogen auf $c_{O\alpha}$ = const). Bei $u_{O_2}$ = 75 % ist z.B. $\overline{c_{O_{ln}}} \approx 2 \ c_{O\omega}$ /9/.

Die Rezirkulation des Gases ist also beim Schlaufenreaktor (wie beim Rührkessel) stärker gegenüber der Rohrcharakteristik der Blasensäule.

- Wie in Beispiel 1 der Styrolpolymerisation sollte auch hier nur das gewollte Produkt in $\dot{M}_{Lab}$ austreten, im beispielsweise betrachteten SCP-System also nur Brühe mit der gewollten Biomasse, nicht aber Substrat (Methanol), wie das beim Schlaufenreaktor mit intensiver Rückmischung der Fall ist. Mit ausgetragenes Substrat belastet auch die spätere Aufarbeitung zur Isolierung des SCP.

Aus den betrachteten Vor- und Nachteilen des Schlaufenreaktors für heterogene Systeme ergibt sich, daß es vorteilhaft ist, auch hier eine Kombination von Schlaufen- und Rohrcharakteristik zu verwirklichen wie sie beispielsweise die Figuren 10 bis 12 zeigen.

Die Erfindung ermöglicht für derartige heterogene Systeme die Kombination von Schlaufenreaktor 8 und Rohrreaktor 9 derart, daß im Schlaufenteil zwar die Flüssigphase ($\dot{M}_{L3}$) rezirkuliert wird, die Gasphase jedoch gemäß Fig. 10 aus dem Schlaufenteil direkt mit $\dot{M}_{G2}$ in den darüber befindlichen Rohrteil 9 aufsteigt ohne Rezirkulation im Schlaufenteil 8 oder gemäß Fig. 11 mit $\dot{M}_{G4}$ direkt und mit $\dot{M}_{G3}$ nach einem Umlauf im Schlaufenteil 8 in den Rohrteil 9 überströmt und in diesem aufsteigt.

Außerdem kann gemäß Fig. 12 dieselbe verfahrensmäßige Koppelung von Schlaufen- und Rohrcharakteristik auch verwirklicht werden, indem der Rohrteil 9 (Blasensäule) um den Schlaufenteil 8 herum angeordnet wird. Anstelle des hier gezeigten Überlaufs zur Erzielung möglichst gasfreien L-Austrags $\dot{M}_{Lab}$ kann auch bei gleichbleibendem Außendurchmesser des Reaktors ein Gasabweiser 10 in der Ausführung gemäß Fig. 10 verwendet werden.

In jedem Fall durchläuft die Luft vom Eintritt $\dot{M}_{Gzu}$ bis Austritt $\dot{M}_{Gab}$ den Gesamtreaktor nur mit Rohrcharakteristik, so daß sich das große treibende Konzentrationsgefälle

$$\Delta C = \overline{C_{O_{ln}}} - C_K \quad /9,10/ \text{ ergibt.}$$

Dabei bleiben die vorgenannten Vorteile des Schlaufenteils 8 voll erhalten.

Da im Rohrteil 9 auch der Flüssigkeitsdurchsatz ($\dot{M}_{L4}$) Rohrcharakteristik aufweist, kann die Zuführung von Substrat so eingestellt werden, daß es bis zum Austritt am Kopf des Reaktors völlig aufgezehrt und in Biomasse umgesetzt ist.

Falls der stetig zuzuführende Liquidstrom $\dot{M}_{Lzu}$ nicht ausreicht für die erforderliche L-Strahlleistung, kann ein zusätzlicher Liquidstrom $\dot{M}_{L5}$ und/oder $\dot{M}_{L6}$ möglichst gasfrei mit der Zulaufpumpe 11 zur L-Düse zurückgefördert werden.

In Figuren 10 und 11 kann der Durchmesser beider Reaktorteile - ebenso wie in Fig. 6 - verschieden sein je nach gewünschter Verweilzeit und Bauhöhe.

In allen Ausführungsarten ergibt sich bei heterogenen G-L-Systemen im Schlaufenteil 8 auch ein beträchtlicher hydrostatischer Mammutantrieb des Umlaufs. In den Figuren 10 bis 12 verstärkt er den hydrodynamischen L-Strahlantrieb. Anstelle des L-Strahlantriebs könnte auch ein gleichsinnig wirkender hydromechanischer Propellerantrieb verwendet werden. Bei entsprechenden Gasdurchsätzen kann der Umlauf im Schlaufenteil 8 auch allein durch Mammutantrieb bewirkt werden.

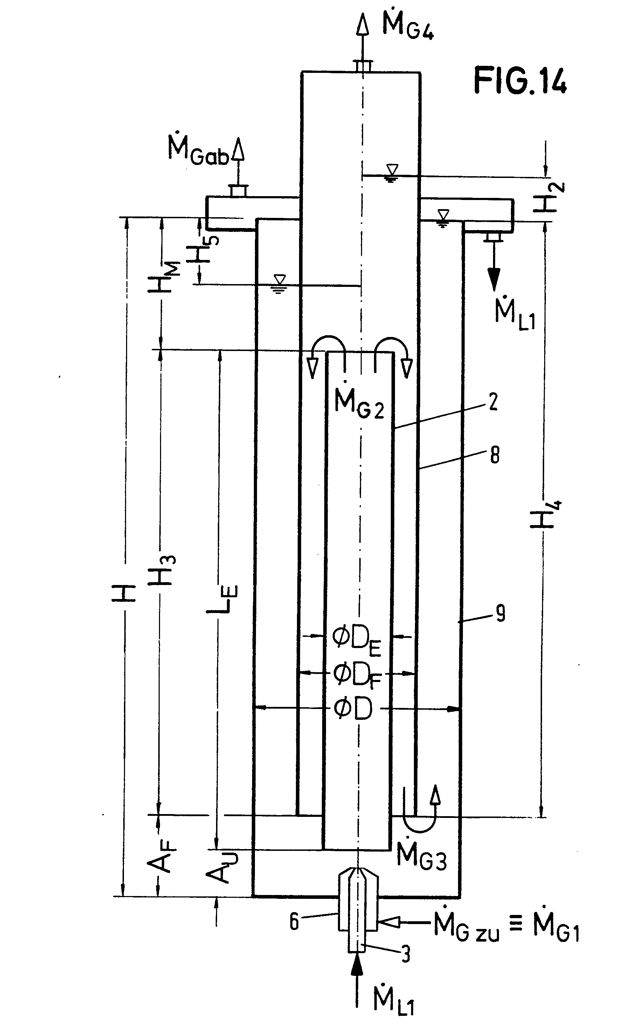

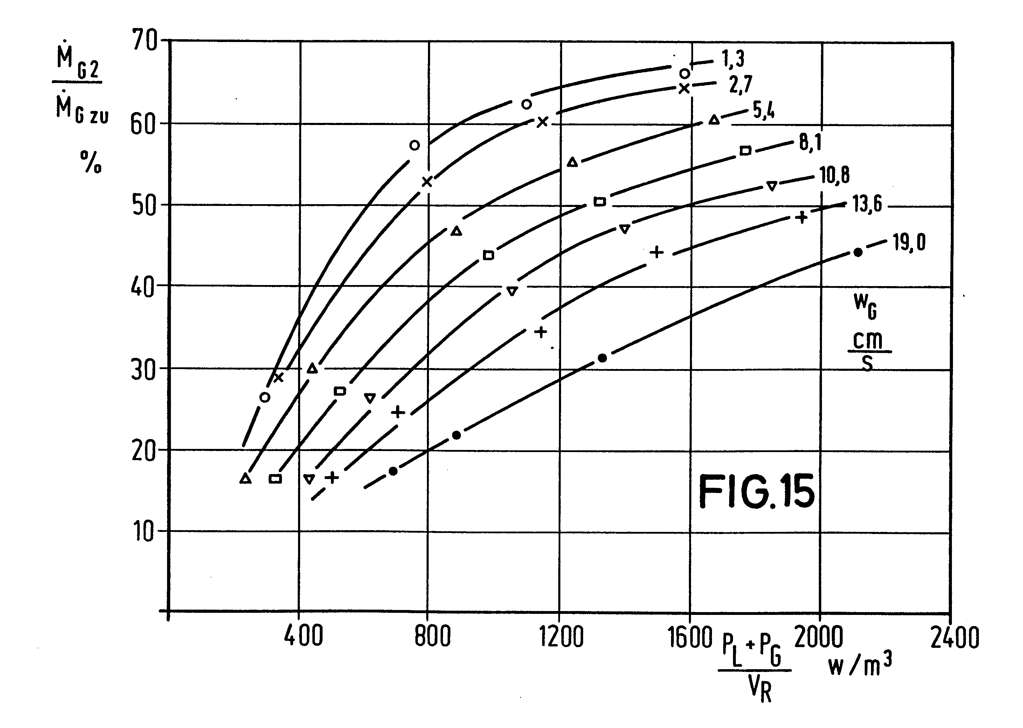

Vorversuche mit dem System Wasser-Luft in einem Reaktor gemäß Figur 14 haben gezeigt (s. Fig. 15), daß bei einem spezifischen Gesamtleistungseintrag mit dem L-Strahl ($P_L$)

und dem Gas ($P_G$) von $\dfrac{P_L + P_G}{V_R} = \dfrac{P}{V_R}$ von $1 - 1,5 \dfrac{kw}{m^3}$
etwa 60 % des zugeführten Gasstroms $\dot{M}_{Gzu}$ als $\dot{M}_{G2}$ von der
Schlaufenströmung eingezogen und zum Rohrteil transportiert
werden können.

Parameter in Fig. 15 ist die "Glasleerrohrgeschwindigkeit"
(s. Fig. 14)

$$W_G = \frac{\dot{M}_{G1}}{\varsigma_G \cdot \frac{\pi}{4} \cdot D_F^2} \qquad (7)$$

Eine Strömungsführung der oberen Umlenkung, wie sie beispielsweise in den Figuren 11 und 12 angedeutet ist, kann
gegenüber den in der Apparatur von Fig. 14 gewonnenen in
Fig. 15 aufgeführten Werten den Anteil des von der abwärts gerichteten Strömung eingezogenen Gasanteils $\dot{M}_{G2}$
sicherlich noch erhöhen, ein Gasabweiser z.B. wie in Fig.
10 ihn hingegen erniedrigen und gegebenenfalls ganz unterbinden.

Es muß je nach Anwendungsfall wirtschaftlich überprüft
werden, ob und wie man den aus dem Schlaufenteil in Fig.
12 , 14 und 16 austretenden Gasstrom $\dot{M}_{G4}$ entweder oben
der abwärts gerichteten Schlaufenströmung oder - wie in
Fig. 12 angedeutet - unten dem Rohrteil zuführt mit Verdichtern beliebiger Art.

Gemäß Fig. 12 können fluide Teilströme z.B. $\dot{M}_{G4}$ aus dem
Schlaufenteil 8 austreten und mit Fördereinrichtung 12 dem
Schlaufenteil $\dot{M}_{G2}$ bzw. dem Rohrteil als $\dot{M}_{G3}$ über einen
Lochring 13 wieder zugeführt werden.

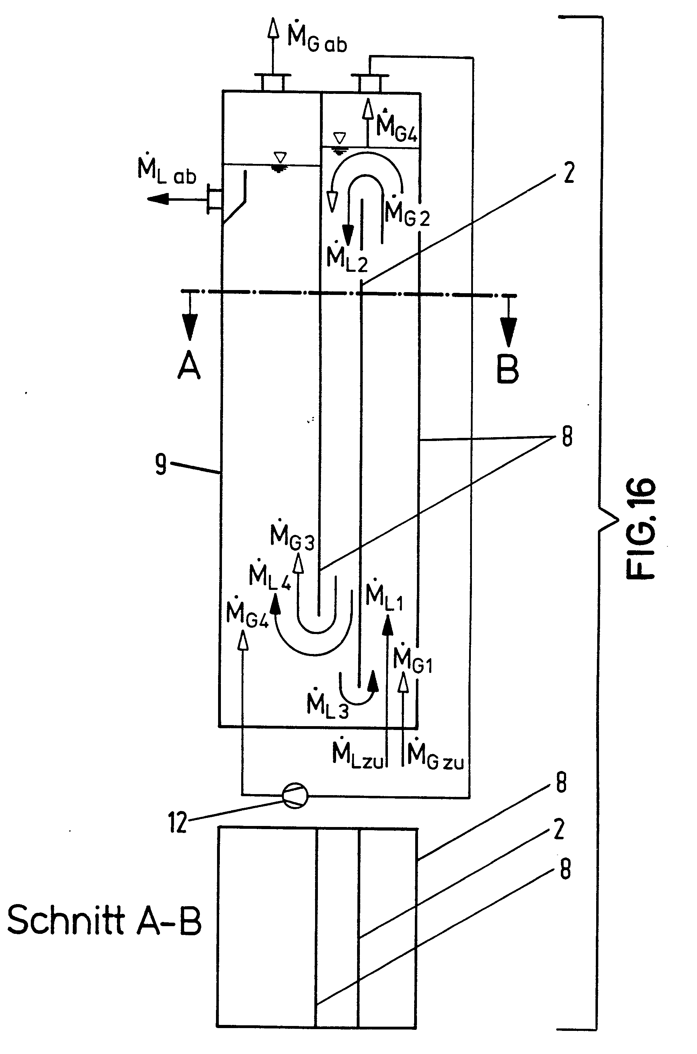

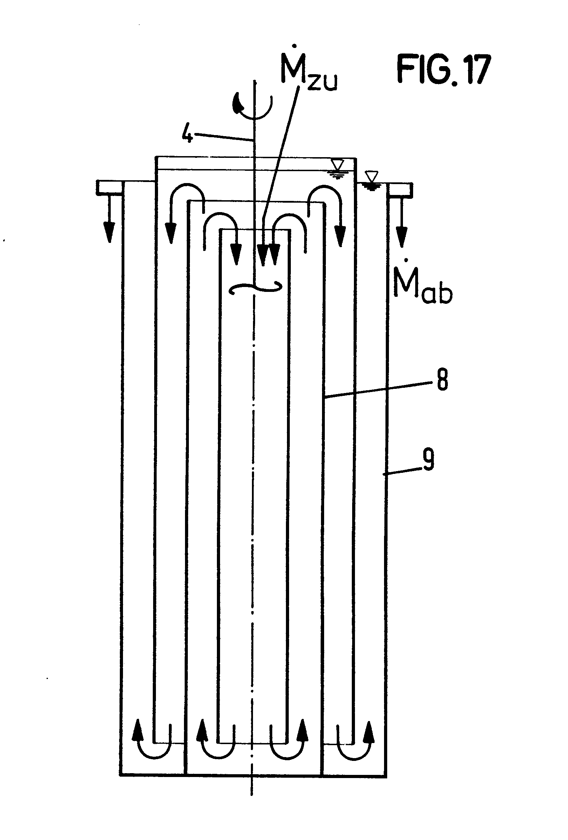

Statt des runden Behälters und der zylindrischen Leitrohre
können auch anders geformte Ausführungen dieselbe Wirkungsweise erreichen, z.B. gemäß Fig. 16 ein rechteckiger Behälter mit ebenen Leitblechen. Schließlich können auch mehrere Rohrteile um den Schlaufenteil herum angeordnet werden, wie es im Prinzip Fig. 17 zeigt.

Als Beispiele für günstige Anwendungen der Erfindung wurden hier ein chemischer und ein biochemischer Prozeß gewählt.

Im ersten Beispiel könnte das gebildete Polymere auch in einem Lösungsmittel unlöslich sein (wie z.B. bei der Styrol-Acrylnitril-Mischpolymerisation in Äthylbenzol) und in Form fester Partikel "ausgefällt" werden. Das Beispiel steht also zugleich für verwandte verfahrenstechnische Grundoperationen wie Fällung, Flockung, Kristallisation. Auch das Monomere könnte in der Trägerflüssigkeit unlöslich sein, wie z.B. bei Emulsionspolymerizationen.

Im zweiten Beispiel werden nur die durch die Erfindung günstig beeinflußten verfahrenstechnischen Grundoperationen Absorption und Diffusion betrachtet unabhängig von der biochemischen Reaktion, mit der sie gekoppelt sind. Das heißt, das zweite Beispiel impliziert auch die Anwendung der Erfindung auf verfahrenstechnische Grundoperationen in homogenen oder heterogenen fluiden Systemen ohne gekoppelte (bio-)chemische Reaktion wie z.B. Absorption zur Gaswäsche oder Gastrennung. Der Absorption von Gas in der L-Phase entspricht aber prinzipiell auch die Lösung eines Feststoffs oder einer Flüssigkeit in der L-Phase, wie sie z.B. für Solventextraktion bestimmend ist.

Im Falle der Kristallisation wird z.B. im Schlaufenteil die Keimbildung und im nachgeschalteten Rohrteil gleichmäßiges Kristallwachstum gefördert.

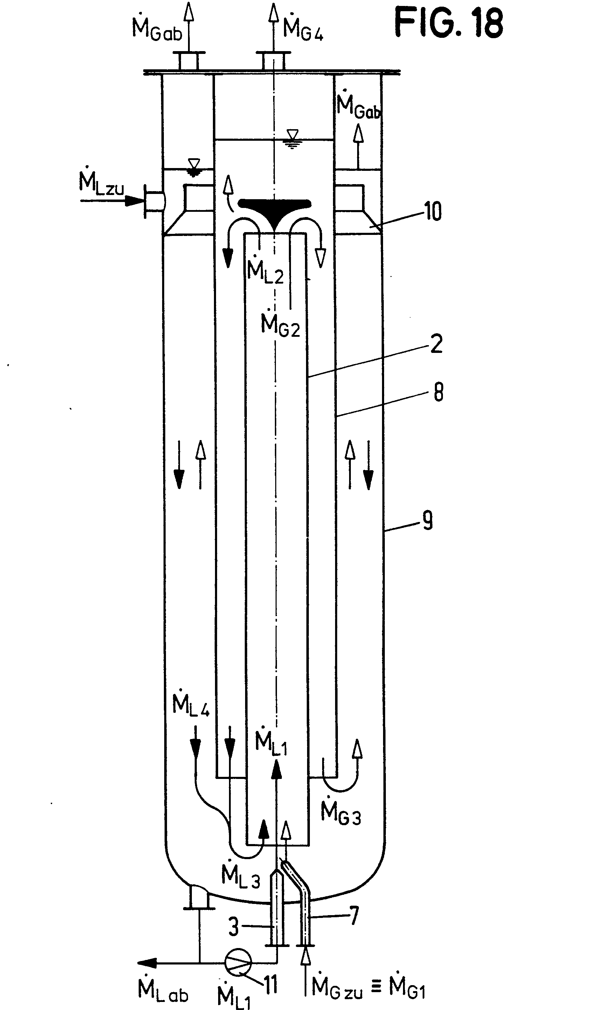

Bei der Absorption zur Gastrennung ist es vorteilhaft, gemäß Fig. 18 im Rohrteil die Richtung der Liquidströmung umzukehren, um hier Liquid und Gas im Gegenstrom zueinander zu führen und damit in bekannter Weise die Stoffübertragung zu verbessern.

- 15 -

0010571

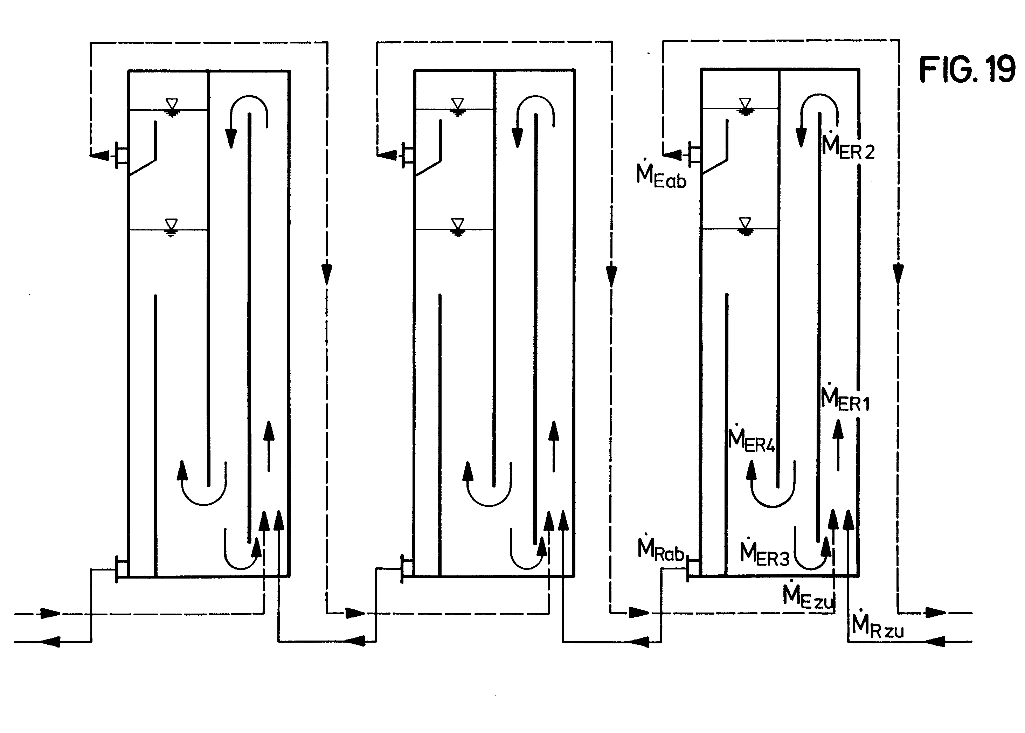

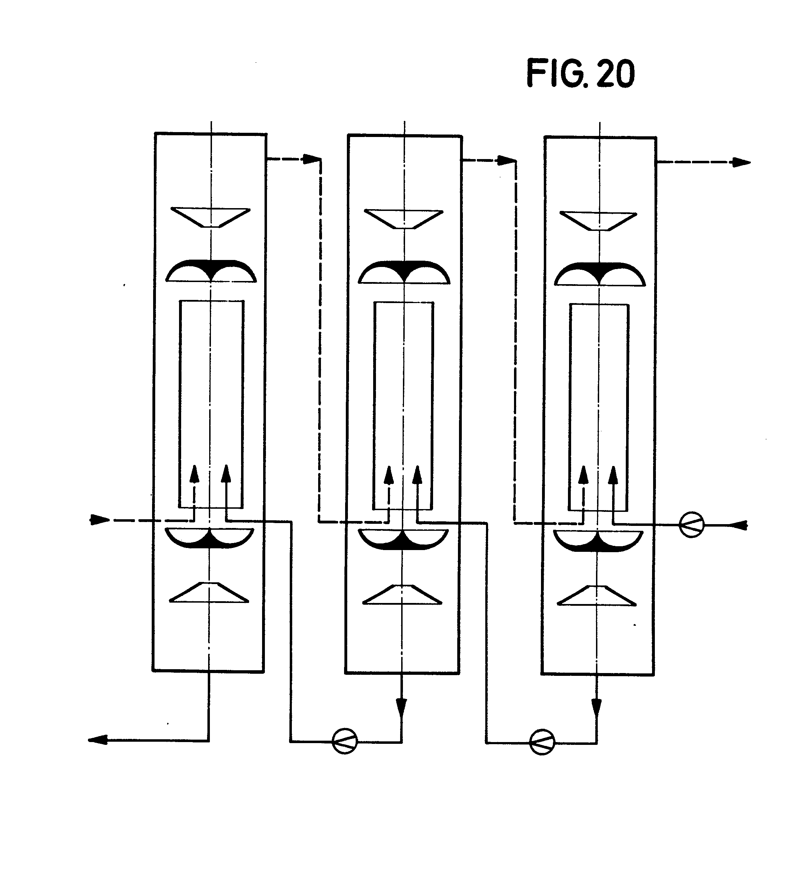

Die Figuren 19 bis 21 und 23 zeigen eine weitere Anwendung der Erfindung am Beispiel der Solventextraktion. Diese Grundoperation dient der Trennung gelöster Komponenten (z.B. der Brenn- und Brutstoffe von Kernkraftwerken) aufgrund von deren unterschiedlicher Löslichkeit (Nerntscher Verteilungskoeffizient") in einer schweren und einer leichten Liquidphase, die nicht miteinander mischbar sind /14/. Die Durchführung dieses Trennverfahrens erfolgt üblicherweise in Gegenstrom-Füllkörper-Kolonnen oder in Misch-Trenn-Batterien (mixer-settler), wie sie Fig. 23 zeigt /14/. Beide Phasen werden z.B. in Rührkesseln (mixer) fein dispergiert, um große L-L-Phasengrenzfläche für die Übertragung der gelösten Stoffe zu schaffen. In nachgeschalteten Absetzkammern (settler) trennen sich die beiden Liquidphasen wieder infolge unterschiedlicher Dichte. Sie durchlaufen resultierend die Misch-Trenn-Batterie im Gegenstrom. Auch hier bringt die Erfindung Vorteile gegenüber der bisherigen Verfahrensweise. Die Erzeugung großer spezifischer L-L-Phasengrenzfläche übernimmt hiernach der Schlaufenteil (vorzugsweise ein Strahl-Schlaufenreaktor) mit den in Beispiel 2 beschriebenen Vorteilen besserer Ver- und Zerteilung disperser Phasen, einfacher Bau- und Betriebsweise und geringerem spezifischen Leistungsaufwand gegenüber dem Rührkessel. Der vor- und/oder nachgeschaltete Rohrteil dient hier als Trennkammer, in der sich die beiden Liquidphasen wegen unterschiedlicher Dichte absetzen und trennen lassen (s. Figuren 19 bis 21). Der Index E steht hier für Extrakt-, R für Raffinat- und ER für Extrakt- und Raffinatphase. Bei Strahlantrieb im Schlaufenteil kann vorteilhafterweise eine L-Phase als Strahl injiziert werden, als Strahlpumpe die andere Phase mit eingetragen und dispergieren und zugleich den Umlauf antreiben, wie Fig. 20 prinzipiell zeigt. Die hintereinander geschalteten Misch-Trenn-Einheiten gemäß Fig. 19 und 20 lassen sich auch in einem Aggregat vereinigen, wie es beispielsweise Fig. 21 zeigt.

0010571

Schließlich gilt die Erfindung auch für homogene Gassysteme, z.B. Gasreaktionen.

Bei allen fluiden Systemen können auch Feststoffe (z.B. als Katalysatoren, Adsorbentien, Ionenaustauscher oder Zer- und Verteilhilfen) in Teilbereichen oder im gesamten Reaktor vorhanden sein, und zwar fluidisiert (z.B. als Wirbelschicht oder als um- und/oder durchlaufendes fluides Teilsystem) oder in Form eines Fest- oder Wanderbettes sowie als Füllkörper oder sonstige Einbauten (z.B. Siebe, Böden).

Literatur:

/1/ Blenke H., K.Bohner, S.Schuster, Beitrag zur optimalen Gestaltung chemischer Reaktoren
Chemie-Ingenieur-Technik, 37 (1965) 3, 289/294

/2/ Blenke H., Schlaufenreaktoren in "Bioreaktoren":
BMFT-Statusseminar "Bioverfahrenstechnik" Braunschweig-Stöckheim 1977, S. 5/44
Herausgeber DFVLR

/3/ Stein W., Zur Berechnung von Schlaufenreaktoren
Chemie-Ingenieur-Technik, 40 (1968) 17, 829/837

/4/ Lehnert J., Berechnung von Mischvorgängen in schlanken Schlaufenreaktoren
Verfahrenstechnik, 6 (1972) 2, 58/63

/5/ Blenke H., Mixing and Dispersing in loop reactors,
Proceeding Int.Symp. on Mixing Faculté
Polytechnique de Mons, 1978

/6/ Blenke H., Einflüsse von Bauform und Wärmeübertragung auf Grenzleistung und Stabilitätsverhalten
chemischer Reaktoren
Chemie-Ingenieur-Technik, 39 (1967) 3, 109/116

/7/ Marquart R., H.Blenke, Umlauf mittel- bis hochviskoser newtonscher und nicht-newtonscher Flüssigkeiten in Propeller-Schlaufenreaktoren;
Umlauf höherviskoser newtonscher und nicht-newtonscher Flüssigkeiten in Strahlschlaufenreaktoren
Verfahrenstechnik 12 (1978) 9 (erscheint demnächst)

/8/ Blenke H., Single Cell Protein (SCP) - Ein industriell produzierter Rohstoff für Nahrungsmittel
VDI-Berichte, Nr. 277 (1977) 127/136

/9/ Blenke H., W.Hirner, Stoffübergang in Gas/Füssigkeits-Strahlreaktoren und Blasenkolonnen
VDI-Berichte, Nr. 218 (1974( 549/578

0010571

/10/ Hirner W., H.Blenke, Gasgehalt und Phasengrenzfläche
in Schlaufen- und Strahlreaktoren
Verfahrenstechnik, 11 (1977) 5

/11/ Fitzer E., W.Fritz, Technische Chemie
Springer-Verlag, Berlin-Heidelberg-New York, 1975

/12/ Raible G., Wärmeübergang im Schlaufenreaktor für
das System Gas/Flüssigkeit
Diss. Universität Stuttgart, 1976

/13/ Blenke H., K.Bohner, W.Pfeiffer, Hydrodynamische
Berechnung von Schlaufenreaktoren für Einphasensysteme
Chemie-Ingenieur-Technik, 43 (1971) 1 + 2, 10/17

/14/ Blenke H., Ingenieuraufgaben in der Kerntechnik
technica, Nr. 5 und 6 (1961), 291 - 296 und 355 -
361

Patentansprüche:

1. Verfahren für (bio-)chemische Stoffumwandlungen (Reaktionen und verfahrenstechnische Stoffbehandlungen (Grundoperationen) in homogenen und heterogenen fluiden Systemen dadurch gekennzeichnet, daß der ein Verfahrensteil (Schlaufenteil) mit Schlaufencharakteristik, die durch Beeinflußung der Rezirkulation zwischen idealer Rührkessel- und realer Rohrcharakteristik eingestellt werden kann und ein weiterer örtlich oder zeitlich vor- und/oder nachgeschalteter Verfahrensteil (Rohrteil) mit Rohrcharakteristik miteinander gekoppelt sind.

2. Verfahren nach Anspruch 1 dadurch gekennzeichnet, daß in homogenen und in heterogenen fluiden Systemen in dem Schlaufenteil fluide Teilsysteme (z.B. Flüssigkeit mit gelösten oder dispergierten Fest- und/oder Liquidkomponenten) einstellbar rezirkuliert werden, andere fluide Teilsysteme (z.B. ein Gasgemisch) hingegen diesen Verfahrensteil mit Rohrcharakteristik durchströmen und daß im Rohrteil alle fluiden Teilsysteme sich mit Rohrcharakteristik im Gleich- oder Gegenstrom zueinander bewegen.

3. Verfahren nach Ansprüchen 1 und 2 dadurch gekennzeichnet, daß z.B. gemäß Fig. 10 aus dem Schlaufenteil fluide Teilströme (hier Gas als $\dot{M}_{G2}$) direkt in den Rohrteil eintreten; oder z.B. gemäß Fig. 11 fluide Teilströme (z.B. $\dot{M}_{G4}$) direkt in den Rohrteil eintreten, andere fluide Teilströme (z.B. $\dot{M}_{G2}$) aber den Umlauf des Schlaufenteils so weit durchlaufen, bis sie (z.B. als $\dot{M}_{G3}$) vor der Rezirkulation aus dem Schlaufenteil in den Rohrteil übergehen; oder daß z.B. gemäß Fig. 12 fluide Teilströme (z.B. $\dot{M}_{G4}$) aus dem Schlaufenteil austreten und mit Fördereinrichtungen dem

Schlaufenteil (wie $\dot{M}_{G2}$) bzw. dem Rohrteil (wie $\dot{M}_{G3}$) z.B. über den in Fig. 12 angedeuteten Lochring wieder zugeführt werden, während andere fluide Teilströme (z.B. $\dot{M}_{G2}$) wie zuvor den Schlaufenteil so weit durchlaufen, bis sie (z.B. als $\dot{M}_{G3}$) vor der Rezirkulation in den Rohrteil übergehen.

4. Verfahren nach Ansprüchen 1 und 2 dadurch gekennzeichnet, daß in heterogenen fluiden Systemen Schlaufenteile zum Zerteilen der dispersen Phasen und zum Verteilen aller Komponenten der dispersen Phasen und zum Verteilen aller Komponenten Rohrteile hingegen zur Verbesserung des (bio-)chemischen Umsatzes sowie zur Wärme- und/oder Stoffübertragung zwischen Teilsystemen oder zu deren Trennung dienen.

5. Verfahren nach Ansprüchen 1 bis 4 dadurch gekennzeichnet, daß in Schlaufen- und/oder Rohrteilen ganz oder teilweise Feststoffe und/oder unlösliche Flüssigkeiten, und zwar fluidisiert (z.B. als Wirbelschicht oder als um- und/oder durchlaufendes fluides Teilsystem), oder Feststoffe als Fest- oder Wanderbett, als Füllkörper, als feste oder bewegte Einbauten (z.B. Siebe, Böden, Rührer, Vibratoren) verwendet werden zur Beeinflussung der Reaktionen (z.B. als Katalysatoren) oder der Grundoperationen (z.B. als Absorbentien, Ionenaustauscher, Extraktionsmedium) oder der Zer- und/oder Verteilwirkung.

6. Vorrichtung zur Durchführung der Verfahren nach Ansprüchen 1 bis 5 dadurch gekennzeichnet, daß Schlaufen- und Rohrteile in einem Apparat vereinigt sind, wobei Rohrteile über und/oder unter Schlaufenteilen z.B. gemäß Fig. 6, 10, 11 und 20 oder um Schlaufenteile herum bzw. neben diesen z.B. gemäß den Fig. 7, 12, 16 bis 19 und 21, oder in Kombination der vorgenannten Arten angeordnet sind.

7. Vorrichtung zur Durchführung der Verfahren nach Ansprüchen 1 bis 5 dadurch gekennzeichnet, daß zur Nutzung der Leitelemente als Zusatzwärmeübertrager (z.B. als Doppelmantelrohre) die "Grenzgröße" des Apparates ohne äußeren Wärmeübertragungskreislauf auf ein Vielfaches (z.B. das 30-fache) üblicher Rührkessel erhöht werden kann.

8. Vorrichtung zur Durchführung der Verfahren nach Ansprüchen 1 bis 5, dadurch gekennzeichnet, daß die Unterkante der Leitvorrichtung 8 z.B. wie in den Figuren 11, 12, 14, 16 und 18 so viel höher als die Unterkante der Leitvorrichtung 2 angeordnet ist, daß im Schlaufenteil des Apparates fluide Teilsysteme (z.B. Flüssigkeit, Lösungen oder Suspensionen) rezirkuliert werden, andere fluide Teilsysteme (z.B. Gasgemische) hingegen vor der Rezirkulation in den Rohrteil überströmen.

9. Vorrichtung zur Durchführung der Verfahren nach Ansprüchen 1 bis 8 dadurch gekennzeichnet, daß fluide Teilströme z.B. gemäß Fig. 10 (hier der Gasstrom $\dot{M}_{G2}$) oder gemäß Fig. 11 (hier der Gasstrom $\dot{M}_{G4}$) direkt aus Schlaufenteilen in Rohrteile eintreten.

10. Vorrichtung zur Durchführung der Verfahren nach Ansprüchen 1 bis 9 dadurch gekennzeichnet, daß z.B. gemäß Fig. 12, 14, 16 und 18 austretende fluide Teilströme (hier der Gasstrom $\dot{M}_{G4}$) und/oder weitere fluide Teilströme (z.B. Frischgas oder Inertgas) zurückgefördert und/oder eingetragenen werden in Schlaufen- und/oder Rohrteile wie z.B. in Fig. 12.

11. Vorrichtung zur Durchführung der Verfahren nach Ansprüchen 1 bis 10 dadurch gekennzeichnet, daß die Umlenkung der Strömung um die Stirnkanten der Leitvorrichtung durch Stromführungen und/oder Gasabweiser beeinflußt wird sowohl hinsichtlich des Druckverlustes als auch hinsichtlich der Trennung von fluiden Teilsystemen, wie z.B. die Fig. 6, 10, 11 und 12, sowie Abb. 4 in /13/ zeigen.

12. Vorrichtung zur Durchführung der Verfahren nach Ansprüchen 1 bis 11 dadurch gekennzeichnet, daß der Apparat auch andere als kolonnenartige Bauformen, z.B. rechteckige, haben kann und als Strömungsleiteinrichtungen auch andere als Rohre , z.B. Leitbleche verwendet werden können wie es z.B. die Fig. 16, 19 und 21 zeigen, und daß die Leiteinrichtungen auch unterteilt und/oder durchbrochen (z.B. geschlitzt oder gelocht) sein können.

13. Vorrichtung zur Durchführung der Verfahren nach Ansprüchen 1 bis 12 dadurch gekennzeichnet, daß die Schlaufen- und Rohrteile z.B. gemäß den Figuren 6, 10, 11 und 20 auch unterschiedliche Querschnittsflächen und Formen haben können.

14. Vorrichtung zur Durchführung der Verfahren nach Ansprüchen 1 bis 13 dadurch gekennzeichnet, daß bei Fließbetrieb z.B. gemäß den Figuren 11 und 12 zuzuführende fluide Teilströme (z.B. $\dot{M}_{Lzu}$) und/oder rückgeführte fluide Teilströme (z.B. $\dot{M}_{L5}$ und/oder $\dot{M}_{L6}$) und/oder inerte fluide Teilströme zur Erzeugung der Strömung und/oder Dispergierung in den Apparat eingebracht werden.

15. Vorrichtung zur Durchführung der Verfahren nach Ansprüchen 1 bis 14 dadurch gekennzeichnet, daß Schlaufen- und Rohrteile getrennt, wie z.B. gemäß Figuren 19 und 20, oder in einem Aggregat vereinigt, wie z.B. gemäß Fig. 21 in beliebiger Reihenfolge hintereinander oder auch parallel geschaltet werden, wobei verschiedene fluide teilströme das Gesamtaggregat im Gleich-, Gegen- oder Kreuzstrom, auch in verschiedenen Kombinationen, durchlaufen können und daß durch den Antrieb des Umlaufs oder durch den Umlauf in Schlaufenteilen ander fluide Teilsysteme eingetragen werden können, wie im Prinzip Fig. 20 zeigt.

16. Vorrichtung zur Durchführung der Verfahren nach Ansprüchen 1 bis 15 dadurch gekennzeichnet, daß die Strömungen im Apparat (z.B. die Umlaufströmungen in Schlaufenteilen gemäß den Figuren 1 bis 5) erzeugt werden durch hydrostatischen (z.B. Mammutpumpe), hydromechanischen (z.B. Propellerpumpe) oder hydrodynamischen (z.B. Strahlförderer) Antrieb, wobei auch mehrere gleich- und/oder verschiedenartige Antriebe neben- und/oder hintereinander gleich- und/oder gegensinnig angeordnet werden können.

17. Verfahren zum Durchführen (bio-)chemischer Reaktionen und verfahrenstechnischer Grundoperationen in homogenen und heterogenen fluiden Systemen, dadurch gekennzeichnet, daß die fluiden Systeme in einer Schlaufe, deren Charakteristik zwischen realer Rohrcharakteristik und idealer Rührkesselcharakteristik einstellbar ist, mit örtlich und zeitlich vor- und/oder nachgeschaltetem Rohr, dessen Strömungsverlauf Rohrcharakteristik aufweist, geführt werden.

18. Verfahren nach Anspruch 17, dadurch gekennzeichnet, daß ein Teilsystem des fluiden Systems mit zwischen idealer Rührkessel- und realer Rohrcharakteristik einstellbarer Charackteristik und das andere Teilsystem des fluiden Systems mit Rohrcharakteristik durch die Schlaufe geleitet werden und im Rohr alle fluiden Teilsysteme mit Rohrcharakteristik gegeneinander oder im Gleichstrom geführt werden.

0010571

19. Verfahren nach den Ansprüchen 17 und 18, dadurch gekennzeichnet, daß Teile eines Teilsystems des fluiden Systems nach Durchlaufen eines Teiles der Schlaufe und andere Teile des Teilsystems nach Durchlaufen der vollständigen Schlaufe in das Rohr geleitet werden.

20. Verfahren nach den Ansprüchen 17 bis 19, dadurch gekennzeichnet, daß Teile des fluiden Systems nach durchlaufen des Rohres in die Schlaufe zurückgeführt werden.

21. Verfahren nach den Ansprüchen 17 bis 20, dadurch gekennzeichnet, daß den fluiden Systemen Feststofe zugesetzt werden.

22. Verfahren nach den Ansprüchen 17 bis 20, dadurch gekennzeichnet, daß den fluiden Systemen unlösliche Flüssigkeiten zugesetzt werden.

23. Vorrichtung zum Durchführen der Verfahren nach den Ansprüchen 17 bis 22, dadurch gekennzeichnet, daß einem Reaktor, in dem Einrichtungen zum Erzeugen von wenigstens einer Schlaufe angeordnet sind, ein Rohr, dessen Strömungsverlauf Rohrcharakteristik aufweist, örtlich und zeitlich vor- und/oder nachgeschaltet ist.

24. Vorrichtung nach Anspruch 23, dadurch gekennzeichnet, daß die Einrichtung zum Erzeugen von wenigstens einer Schlaufe und/oder die Reaktorwände als Wärmetauscher ausgebildet sind.

0010571

FIG.1

FIG.2

# FIG.3

# FIG.4

# FIG.5

0010571

FIG. 6

$\dot{M}_{zu} \equiv \dot{M}_1$

$\dot{M}_2$

$\dot{M}_3$

8

2

9

$\dot{M}_1$

$\dot{M}_{ab} \equiv \dot{M}_1$

0010571

FIG. 7

0010571

FIG. 8

**FIG. 9**

Gas | Liquid

Film | Kern

$c_G$

$c_0 = \dfrac{c_G}{He}$

$c = f(x)$

$c_K$

$0 \qquad x = \delta \qquad \longrightarrow \; x$

FIG.10

7/19

0010571

0010571

**FIG. 11**

0010571

FIG. 12

0010571

FIG. 13

FIG.14

FIG.15

0010571

$\dot{M}_{Gab}$

$\dot{M}_{G4}$

$\dot{M}_{Lab}$

$\dot{M}_{G2}$

$\dot{M}_{L2}$

2

A

B

8

9

$\dot{M}_{G3}$

$\dot{M}_{L4}$

$\dot{M}_{L1}$

$\dot{M}_{G4}$

$\dot{M}_{G1}$

$\dot{M}_{L3}$

$\dot{M}_{Lzu}$  $\dot{M}_{Gzu}$

12

8

2

8

Schnitt A–B

FIG.16

FIG.17

0010571

15 / 19

FIG. 18

FIG.19

0010571

FIG. 20

FIG. 21

FIG. 22

FIG. 23

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| | DE - C - 736 284 (I.G. FARBENINDUS-TRIE)<br><br>* Seite 2, Zeilen 6-106; Abbildungen 1,2 *<br><br>-- | 1,2,4-8,16,17,21,23,24 |
| | US - A - 3 458 467 (K. HERRLE et al.)<br><br>* Zusammenfassung; Spalte 2, Zeile 34 - Spalte 3, Zeile 37; Spalte 4, Zeilen 7-21; Abbildungen 4,5,7 *<br><br>-- | 1,4,15-17,19,23,24 |
| | US - A - 3 775 302 (J. KUBO et al.)<br><br>* Abbildung; Zusammenfassung; Spalte 2, Zeile 50 - Spalte 5, Zeile 46 *<br><br>-- | 1-6,9,11,14,16,17 |
| | FR - A - 1 220 538 (FARBWERKE HOECHST)<br><br>* Seite 5, Spalte 1, Zeile 51 - Spalte 2, Zeile 36; Abbildungen 1,2 *<br><br>-- | 1,4,6,7,9,10,23,24 |
| A | DE - A - 2 165 742 (NIPPON OIL) | |
| A | DE - A - 2 030 393 (NIPPON OIL) | |
| A | DE - A - 2 453 869 (NIPPON OIL) | |
| A | US - A - 3 763 266 (M.L. HENDERSON) | |
| A | FR - A - 2 018 420 (BASF) | |
| A | GB - A - 835 926 (U.K.A.E.) | |
| | ./. | |

### KLASSIFIKATION DER ANMELDUNG (Int.Cl. 3)

B 01 J   8/20
          8/22
        10/00
        14/00
        19/24
C 12 M   1/08//
B 01 D  11/04
C 08 F   2/00
        12/00

### RECHERCHIERTE SACHGEBIETE (Int. Cl. 3)

B 01 J   8/20
          8/22
        10/00
        14/00
        19/24
        47/10
B 01 D  11/04
C 02 F   3/22
C 22 B   3/02
C 08 F   2/00
         2/02
       12/08
C 12 M   1/08

### KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

Der vorliegende Recherchenbericht wurde für alle Patentansprüche erstellt.

| Recherchenort | Abschlußdatum der Recherche | Prüfer |
|---|---|---|
| Den Haag | 11-02-1980 | SIEM |

EPA form 1503.1  06.78

| | EINSCHLÄGIGE DOKUMENTE | | KLASSIFIKATION DER ANMELDUNG (Int.Cl. 3) |
|---|---|---|---|
| Kategorie | Kennzeichnung des Dokuments mit Angabe, soweit erforderlich, der maßgeblichen Teile | betrifft Anspruch | |
| A | FR - A - 2 156 376 (B.A.S.F.) | | |
| A | US - A - 3 439 807 (M. DANJES) | | |
| A | FR - A - 522 041 (V. LAMY) | | |
| A | FR - A - 1 529 536 (I.F.P.) | | |
| A | SU - A - 171 366 (A.U. MAMUNYA et al.) | | |
| E | EP - A - 0 003 548 (HOECHST) (Anmeldetag: 30-01-1979)(Veröffentlichungstag: 22-08-1979) ---- | | RECHERCHIERTE SACHGEBIETE (Int. Cl. 3) |